# EUROPEAN PATENT APPLICATION

(11) **EP 2 420 287 A1**
(43) Date of publication of application: **22.02.2012**
(21) Application number: 11178208.2
(22) Date of filing: 19.08.2011
(51) Int. Cl.: A61N 5/06, A61C 19/06

(54) **Method for enhancing viability of periodontal tissue cells**

(30) Priority: 20.08.2010 TW 099128039
(71) Applicant: Ho, Ming- Hua, Tapei City 100 (TW); Hong, Lu-Sheng, Taipei City 10660 (TW); Jetts Technology Co., Ltd., Xindian City T'ai pei 231 (TW)
(72) Inventor: Ho, Ming-Hua, 100 Tapei City (TW); Hong, Lu-Sheng, 10660 Tapei City (TW); Yu, Hsian-Shin, 231 Xindian City, Tapei (TW)
(74) Representative: Reichert, Sabine

(57) **Abstract**

In a method for enhancing viability of periodontal tissue cells associated with more than one tooth, the periodontal tissue cells are irradiated simultaneously by an LED (light emitting diode) module having a light emitting range covering the associated teeth, wherein the irradiating energy is between 0.1 J/cm² and 10 J/cm².

## Description

The present invention relates to home care for teeth, and more particular to a method for enhancing viability of periodontal tissue cells.

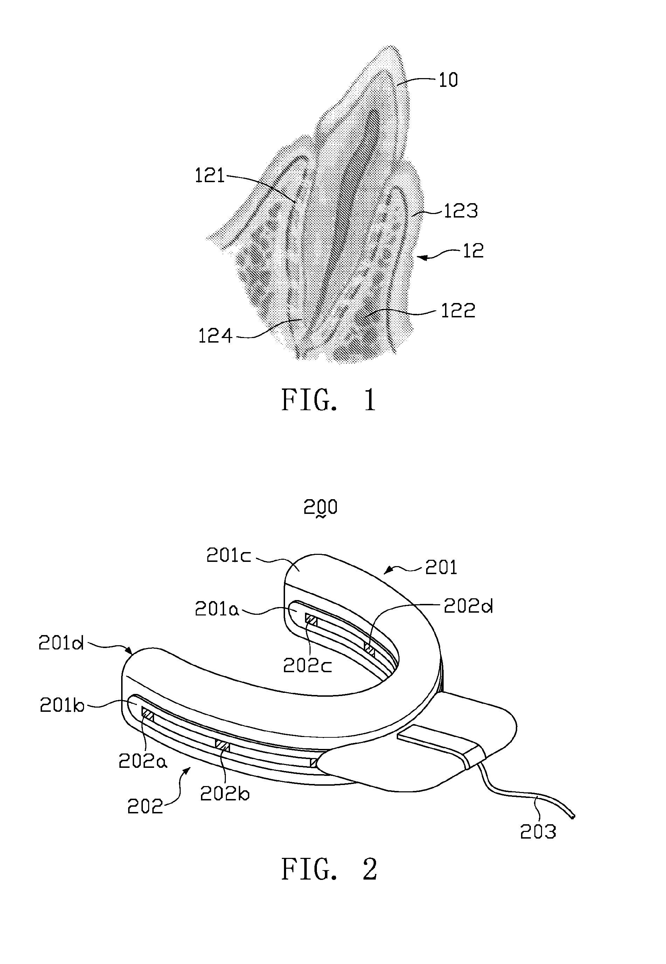

Please refer to FIG. 1, which is a cross-sectional view schematically illustrating a tooth 10 and associated tissues 12 including periodontal ligmant 121, alveolar bone 122, gingivae 123 and cementum 124. The periodontal tissues support and secure the teeth and facilitate the chewing function of the teeth. The periodontal tissues are subject to damage from toxins released by dental plaques residing in dental necks. The damage of periodontal tissues would result in, for example, toothache, suppuration, gingivitis, gingival atrophy, tooth gomphiasis and finally periodontal diseases. In addition, the patient may suffer from problems of shameful looks and annoying sensitivity to temperature and/or stimulative materials.

Common dental therapy aims to kill bacteria and treat inflammation. Invasive surgical curettage accompanied by administration of antibiotics and/or antiinflammatory medicines might be required, and a variety of sequelae might thus be rendered.

Laser irradiation is another option or auxiliary therapy for periodontal tissue treatment. The laser irradiation functions for killing bacteria and inhibiting biofilm formation. Due to high energy of the laser irradiation, there exists a risk of damaging teeth, periodontal tissues or other oral tissues. Therefore, laser irradiation should only be executed by professionals in order to well control irradiating target, energy and duration.

For preventing from periodontal diseases, there is a need to provide periodontal maintenance means for daily home care purpose.

Therefore, the present invention provides a method for enhancing viability of periodontal tissue cells, which is feasible for daily home care of teeth.

In one embodiment, the present invention provides a method for enhancing viability of periodontal tissue cells associated with more than one tooth, comprising irradiating the periodontal tissue cells simultaneously by an LED (light emitting diode) module having a light emitting range covering the associated teeth, wherein the irradiating energy is between 0.1 J/cm² and 10 J/cm².

In an embodiment, the periodontal tissue cells may include alveolar osteoblasts, and the irradiating energy is between 0.2 J/cm² and 5 J/cm² when the LED light module emits a blue light having a wavelength of 415±25 nm and between 1 J/cm² and 10 J/cm² when the LED light module emits a yellow light having a wavelength of 575±25 nm or a red light having a wavelength of 635±25 nm.

In another embodiment, the periodontal tissue cells may include gingival fibroblasts, and the irradiating energy is between 1 J/cm² and 10 J/cm² when the LED light module emits a yellow light having a wavelength of 575±25 nm or a red light having a wavelength of 635±25 nm.

In a further embodiment, the periodontal tissue cells may include periodontal fibroblasts, and the irradiating energy is preferably between 1 J/cm² and 5 J/cm² when the LED light module emits a blue light having a wavelength of 415±25 nm, and preferably between 1 J/cm² and 10 J/cm² when the LED light module emits a yellow light having a wavelength of 575±25 nm or a red light having a wavelength of 635±25 nm.

In a still further embodiment, the periodontal tissue cells may include periodontal ligmant fibroblasts, and the irradiating energy is preferably between 0.2 J/cm² and 5 J/cm² when the LED light module emits a blue light having a wavelength of 415±25 nm, and preferably between 0.5 J/cm² and 6 J/cm² when the LED light module emits a yellow light having a wavelength of 575±25 nm or a red light having a wavelength of 635±25 nm.

In an embodiment, the LED module includes a plurality of LED light sources, adjacent two of which have a partially overlapping light emitting rang so that each of the teeth irradiated by the adjacent two LED light sources is subjected to an irradiating energy ranged between 0.1 J/cm² and 10 J/cm².

In another embodiment, the light emitting range has a center at a border of gingivae.

The above contents of the present invention will become more readily apparent to those ordinarily skilled in the art after reviewing the following detailed description and accompanying drawings, in which:
FIG. 1 is a cross-sectional view illustrating a tooth and associated tissues;
FIG. 2 is a schematic diagram illustrating an irradiating module adapted to implementing the irradiation according to an embodiment of the present invention;
FIGS. 3A∼3F are bar charts revealing mitochondria viability changes in a variety of experiments conducted for different periodontal tissue cells;
FIGS. 4A∼4I are bar charts revealing ALPase activity changes in a variety of experiments conducted for different periodontal tissue cells; and
FIGS. 5A∼5F are bar charts revealing cell proliferation capabilities in a variety of experiments conducted for different periodontal tissue cells.

The present invention will now be described more specifically with reference to the following embodiments. It is to be noted that the following descriptions of preferred embodiments of this invention are presented herein for purpose of illustration and description only; it is not intended to be exhaustive or to be limited to the precise form disclosed.

According to the present invention, LED irradiation with a specified optical condition and a specified irradiating manner is used for enhancing viability of periodontal tissue cells while being applicable to daily home care. By enhancing viability of periodontal tissue cells, periodontal tissues are strengthened so as to prevent from diseases. Even if the tissues are damaged, they can readily restore to health by way of the present method.

The term "periodontal tissues" used herein includes alveolar osteoblasts, gingival fibroblasts, periodontal fibroblasts and periodontal ligmant fibroblasts. The viability of all these cells can be enhanced by way of the present method.

Regarding the optical condition for implementing the LED irradiation according to the present invention, irradiation energy effective to viability enhancement and cell regeneration of periodontal tissues is specified.

For home care purpose, the irradiation energy has to be not only effective to viability enhancement and cell regeneration but also harmless to normal cells. According to the present invention, an irradiation energy range between 0.1 J/cm² and 10 J/cm² is recommended. A variety of experiments to be described later support the selection of the irradiation energy. Furthermore, in spite optimal irradiation wavelengths may vary with cells to be strengthened, blue irradiation with wavelengths of 415±25 nm, yellow irradiation with wavelengths of 575±25 nm and red irradiation with wavelengths of 635±25 nm are preferred. In other embodiments of the present invention, combinations of different irradiation wavelengths may be used to enhance viability and regeneration of various cells so as to further improve home care effect.

With regards to the irradiating manner for implementing the LED irradiation according to the present invention, more than one tooth is irradiated simultaneously at a proper position near the periodontal tissues in view of home care effect, efficiency and practicability. On these conditions, the optimal irradiating duration is ranged between 10 seconds and 20 minutes, which is short enough to encourage daily use.

For example, a brace-like irradiating module as illustrated in FIG. 2 can be used for implementing the irradiation. It is to be noted that the brace-like irradiating module is for illustration only, and not intended to be limited thereto. Those skilled in the art may also use other suitable modules to achieve the similar purpose.

FIG. 2 schematically illustrates an LED irradiating device 200 useful for implementing an embodiment of the present invention, which includes a main body 201, a light-emitting member 202 and a power supply unit 203. In this embodiment, the main body 201 has a U-shape configuration flexibly conforming to general dentition so that the user is able to naturally close his mouth while using the device. The main body 201 includes an inner surface 201a, an outer surface 201b and a conjunction surface 201c, which combine to define a space 201d for accommodating maxillary or mandibular teeth. An easy device would be advantageous in encouraging frequent home care use.

The light-emitting member 202 includes at least one LED light source irradiating for more than one teeth at the same time. In the example as illustrated in FIG. 2, at least four LED light sources 202a, 202b, 202c and 202d are mounted onto the inner surface 201a and the outer surface 201b of the main body 201, respectively. Nevertheless, more or less than four LED light sources may be used, depending on practical requirements.

Preferably, the LED light sources have a light emitting range has a center at the border of gingivae so as to efficiently and well irradiate over the periodontal tissues. In an embodiment, the light path emitted by each of the LED light sources may cover a sectorial range, e.g. with an included angle of 120 degrees, so as to simultaneously irradiate more than one tooth. For equalizing the irradiation onto the teeth, every adjacent two of the light sources may be arranged to emit partially overlapping light.

The powers of the LED light sources need not be specifically limited. Nevertheless, the irradiation energy required by the present invention, i.e. about 0.1∼10 J/m², should be complied with in the preferred irradiation duration for home care use, e.g. 10 seconds to 20 minutes, while avoiding damage to teeth, periodontal and other oral tissues.

Even though more than four LED light sources 202a, 202b, 202c and 202d are equipped in the above example, all or partial the light sources can be selectively powered on, depending on practical requirements.

Hereinafter, a variety of experiments following in-vitro irradiation to periodontal tissue cells on specified conditions are described. The periodontal tissue cells include alveolar osteoblasts, gingival fibroblasts, periodontal fibroblasts and periodontal ligmant fibroblasts. The experiments include viability tests of cells and regeneration tests of cells. According to the experimental results, the irradiation condition as well as the viability enhancement of the periodontal tissue cells can be understood.

### VIABILITY TESTS OF CELLS

In the viability tests of cells, the viability of mitochondria and the activity of alkaline phosphatase (ALPase) in periodontal tissue cells are used as indices.

### (1) mitochondria tests

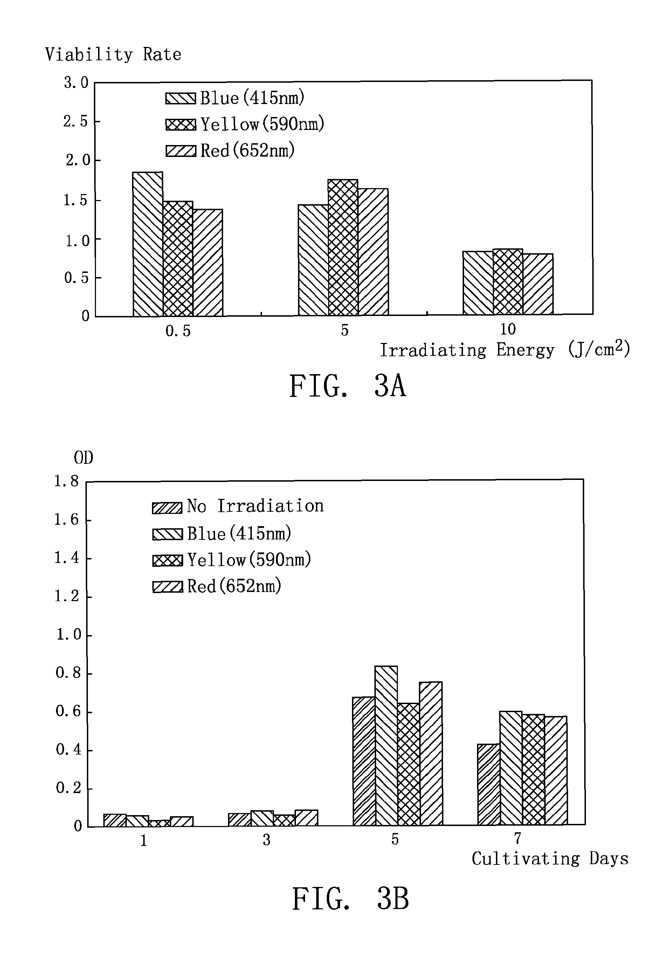

First of all, irradiation is conducted onto each of the four periodontal tissue cells, i.e. the alveolar osteoblasts, gingival fibroblasts, periodontal fibroblasts and periodontal ligmant fibroblasts, with selected wavelengths of LED light and selected irradiating energies. The three selected wavelengths of LED light include substantially 652 nm directing to red LED light, substantially 590 nm directing to yellow LED light, and substantially 415 nm directing to blue LED light. The periodontal tissue cells, after being irradiated, are cultivated for selected days and then analyzed by way of MTT colorimetry assay. An MTT agent, e.g. 3-4,5-dimethylthiazol-2-yl)-2,5-diphenyl tetrazolium bromide, takes part a reduction reaction with succinatedehydrogenase existing in mitochondria of cells in the MTT colorimetry assay. Accordingly, respective viability changes of the alveolar osteoblasts, gingival fibroblasts, periodontal fibroblasts and periodontal ligmant fibroblasts can be measured according to the results of the reduction reactions. The viability changes are summarized in FIGS. 3A∼3E and are described as follows.

In FIG. 3A, the mitochondria viability change of the alveolar osteoblasts, after being irradiated and cultivated for three days, is illustrated. The correlation of viability rate to irradiating energy is schematically shown. In this experiment, three wavelengths of LED light, 415 nm, 590 nm, 652 nm, and three different irradiating energies, 0.5 J/cm², 5 J/cm² and 10 J/cm², are selected for use. The term "viability rate" specified for mitochondria used herein and hereinafter indicates a ratio of averaged mitochondria viability of the alveolar osteoblasts subjected to irradiation (experimental group) to that of the alveolar osteoblasts receiving no irradiation (control group).

According to the experimental data, it can be seen that the mitochondria viability performance of the experimental group is apparently better than that of the control group. The optical condition of 415 nm wavelength and 0.5 J/cm² irradiating energy exhibits a particularly satisfactory result for the alveolar osteoblasts, wherein the viability is almost doubled with irradiation. It can also be seen that with the use of different wavelength of LED light, the optimal irradiating energy may change.

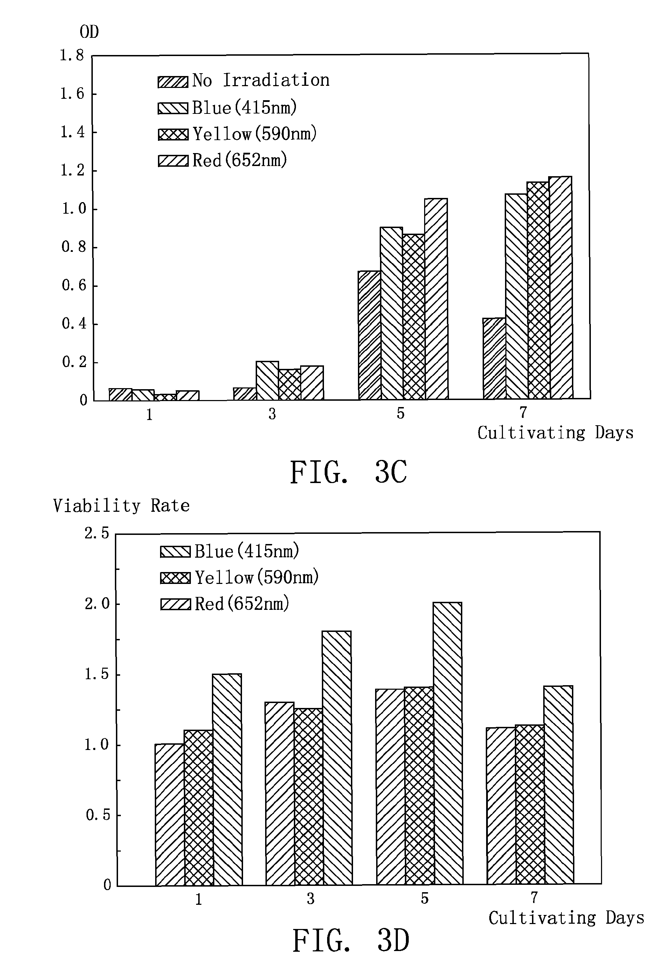

FIG. 3B and FIG. 3C reveal the mitochondria viability performance of irradiated gingival fibroblasts with two different irradiating energies, respectively, by way of correlation of OD (optical density) to cultivation days. The irradiating energy is 2.5 J/cm² in FIG. 3B, and 5 J/cm² in FIG. 3C.

According to the experimental data, it can be seen that the mitochondria viability performance of the experimental group is apparently better than that of the control group. The 5 J/cm² irradiating energy exhibits a particularly satisfactory result for the gingival fibroblasts, wherein the viability is better than doubled with irradiation and 7-day cultivation. It can also be seen that under different irradiating energy conditions, the optimal wavelength of LED light may change.

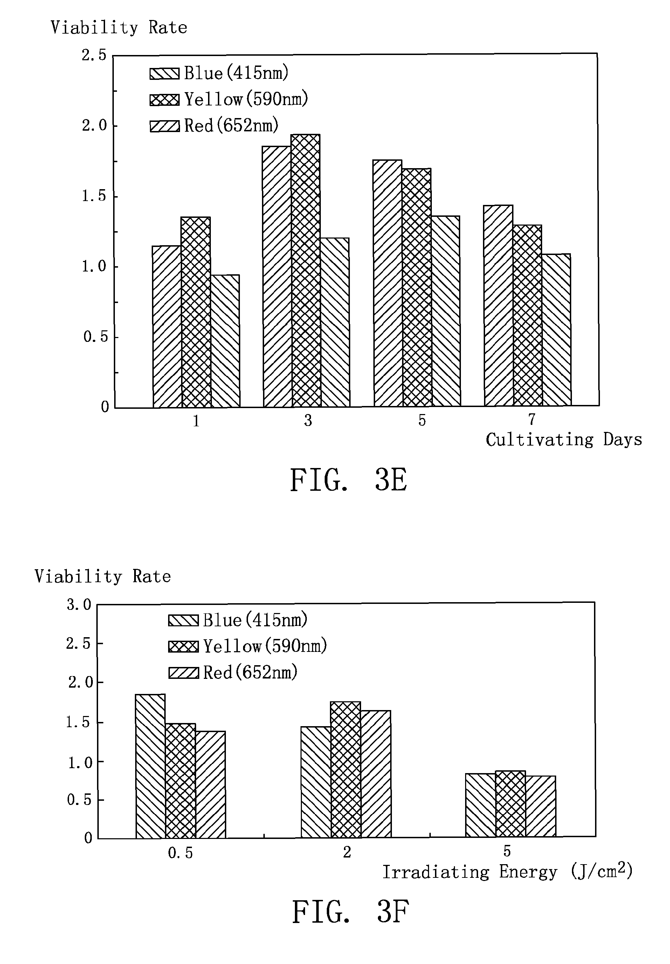

FIG. 3D and FIG. 3E reveal the mitochondria viability performance of irradiated periodontal fibroblasts with two different irradiating energies, respectively, by way of correlation of viability rate to cultivation days. The irradiating energy is 2.5 J/cm² in FIG. 3D, and 5 J/cm² in FIG. 3E.

According to the experimental data, it can be seen that the mitochondria viability performance of the experimental group is apparently better than that of the control group. The viability rate is even doubled on a variety of wavelength and energy combinations. It can also be seen that under different irradiating energy conditions, the optimal wavelength of LED light may change. For example, blue light is the best when 2.5 J/cm² is selected for use, while red light and yellow light are better when 5 J/cm² is selected for use.

FIG. 3F reveals the mitochondria viability change of the periodontal ligmant fibroblasts, after being irradiated and cultivated for three days, is illustrated. The correlation of viability rate to irradiating energy is schematically shown. In this experiment, three wavelengths of LED light, 415 nm, 590 nm, 652 nm, and three different irradiating energies, 0.5 J/cm², 2 J/cm² and 5 J/cm², are selected for use.

According to the experimental data, it can be seen that the mitochondria viability performance of the experimental group is apparently better than that of the control group. The optical condition of 415 nm wavelength and 0.5 J/cm² irradiating energy exhibits a particularly satisfactory result for the periodontal ligmant fibroblasts, wherein the viability is near doubled with irradiation. The optical condition of 590 nm and 5 J/cm² irradiating energy also directs to satisfactory performance. It is thus understood that with the use of different wavelength of LED light, the optimal irradiating energy may change.

### (2) ALPase assay (alkaline phosphatase assay)

First of all, irradiation is conducted onto each of the four periodontal tissue cells, i.e. the alveolar osteoblasts, gingival fibroblasts, periodontal fibroblasts and periodontal ligmant fibroblasts, with selected wavelengths of LED light and selected irradiating energies. The three selected wavelengths of LED light include substantially 652 nm directing to red LED light, substantially 590 nm directing to yellow LED light, and substantially 415 nm directing to blue LED light. The periodontal tissue cells, after being irradiated, are cultivated for selected days and then analyzed by way of ALPase assay.

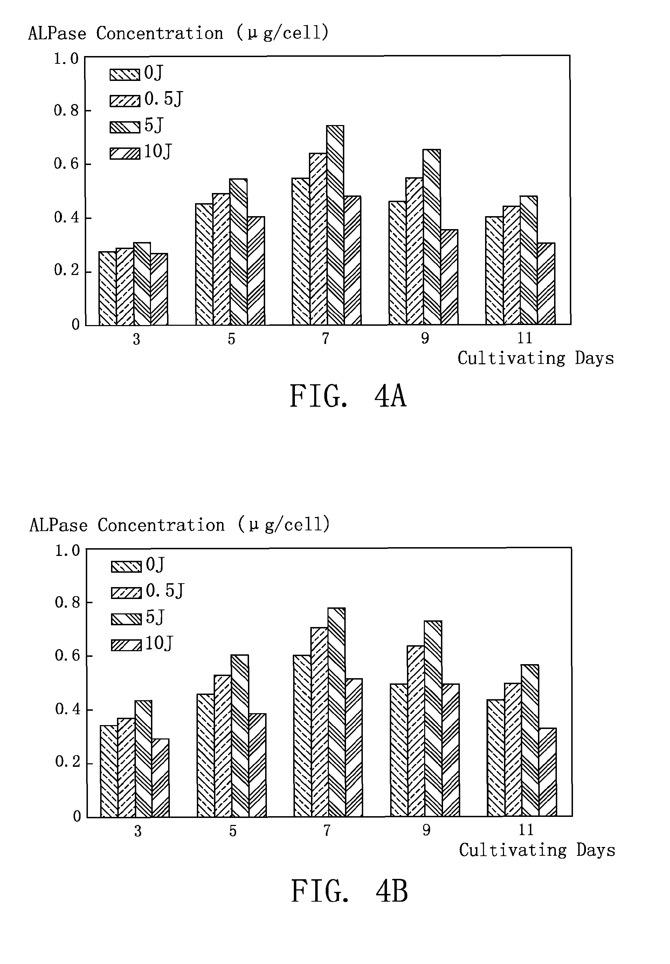

Since ALPase in cells is capable of catalyzing p-nitrophrnylatephosphate reaction, and the absorption peak of the produced p-nitrophrnylate anion is around 405 nm, the ALPase activity can be realized by checking the p-nitrophrnylate anion existing in the cells. The experimental results are summarized in FIGS. 4A∼4I and are described as follows.

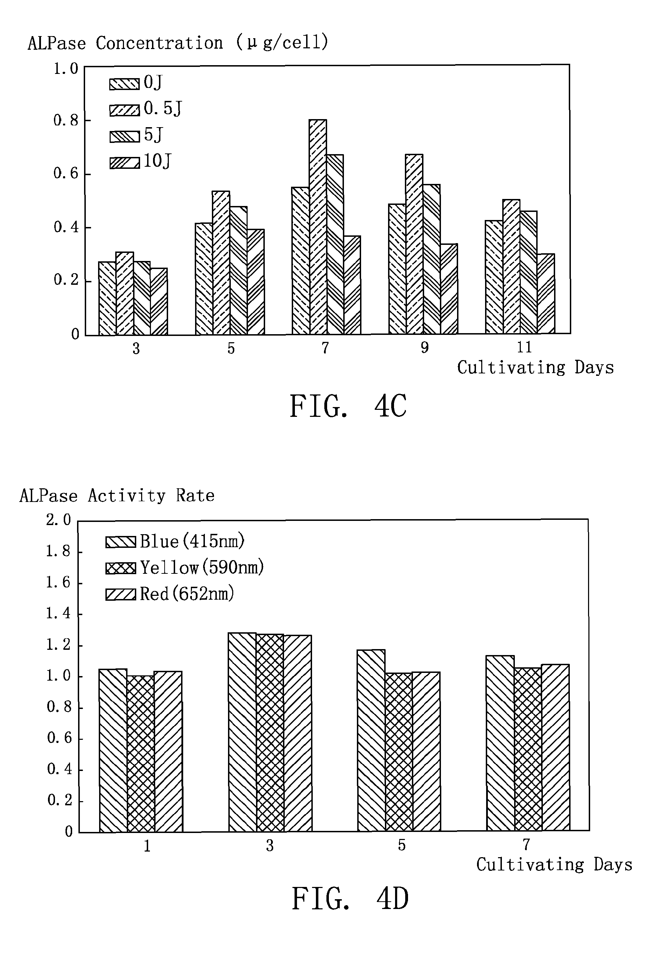

Each of FIGS, 4A, 4B and 4C illustrates the ALPase assay result of the alveolar osteoblasts, after being irradiated with the same wavelength and four different irradiating energies and cultivated for various days. The wavelengths used in FIGS. 4A, 4B and 4C are 652 nm directing to red light, 590 nm directing to yellow light, and 415 nm directing to blue light, respectively. The four irradiating energies includes 0.1 J/cm², 0.5 J/cm², 5 J/cm² and 10 J/cm². The ALPase assay result is indicated by correlation of ALPase concentration (µg/cell) in the alveolar osteoblasts to cultivating days.

It can be seen from FIGS. 4A∼4C that the ALPase activity performance of the experimental group is apparently better than that of the control group, no matter whether blue, yellow or red light is used for irradiation. As shown, when blue light is used (FIG. 4C), irradiating energy of 0.5 J/cm² exhibits particular satisfactory activity enhancing performance. On the other hand, when yellow or red light is used (FIG. 4A or 4B), irradiating energy of 5 J/cm² exhibits better activity enhancing performance.

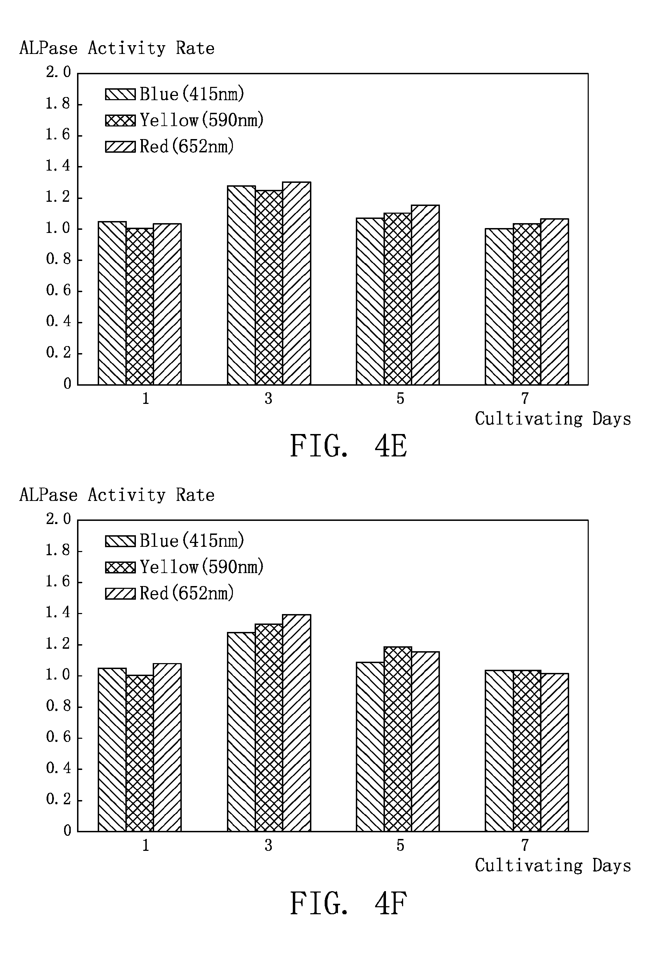

Each of FIGS. 4D, 4E and 4F illustrates the ALPase assay result of the gingival fibroblasts, after being irradiated with the same energy and three different irradiating wavelengths and cultivated for various days. The wavelengths used in FIGS. 4D, 4E and 4F are 415 nm (blue light), 590 nm (yellow light) and 652 nm (red light). The irradiating energies used in the experiments of FIGS. 4D, 4E and 4F are 0.5 J/cm², 2 J/cm² and 5 J/cm², respectively. The ALPase assay result is indicated by correlation of ALPase activity rate to cultivating days. The term "activity rate" specified for ALPase used herein and hereinafter indicates a ratio of averaged ALPase concentration of the gingival fibroblasts subjected to irradiation (experimental group) to that of the gingival fibroblasts receiving no irradiation (control group).

It can be seen from FIGS. 4D~4F that the ALPase activity performance of the experimental group is apparently better than that of the control group, and the irradiation facilitates the secretion of ALPase in cells no matter whether blue, yellow or red light is used for irradiation, and no matter 0.5 J/cm², 2 J/cm² or 5 J/cm² is used as the irradiating energy. Nevertheless, it is understood that with the use of different wavelength of LED light, the optimal irradiating energy may change, and vice versa.

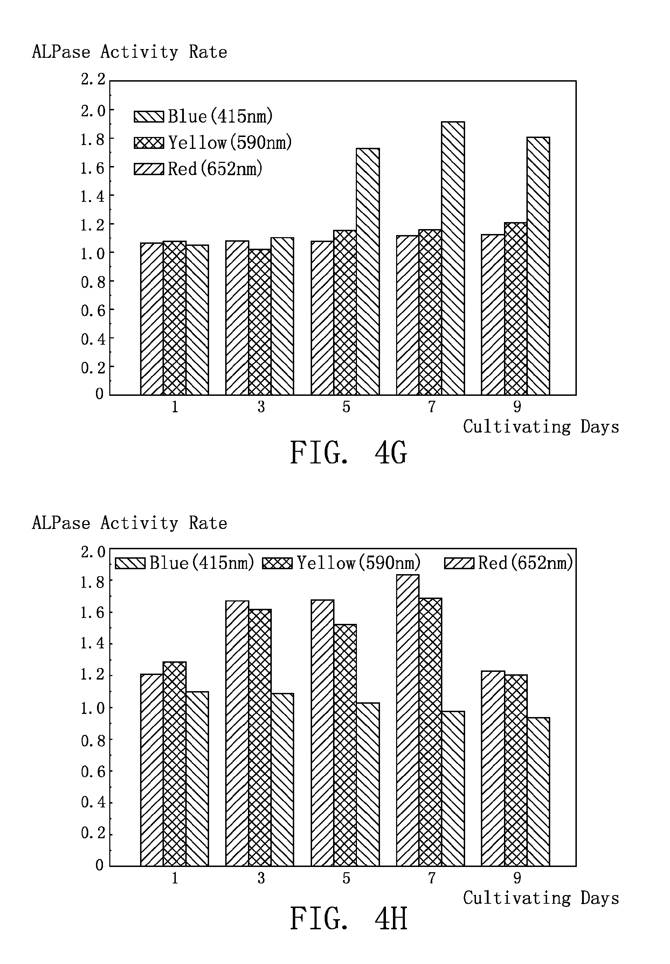

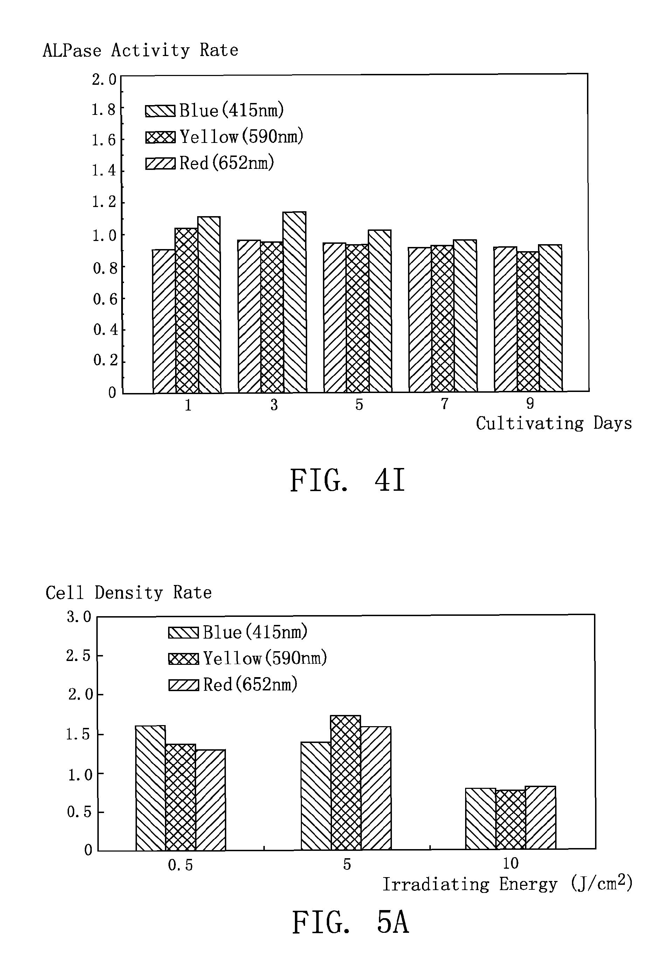

Each of FIGS. 4G, 4H and 4I illustrates the ALPase assay result of the periodontal fibroblasts, after being irradiated with the same energy and three different irradiating wavelengths and cultivated for various days. The wavelengths used in FIGS. 4G, 4H and 4I are 415 nm (blue light), 590 nm (yellow light) and 652 nm (red light). The irradiating energies used in the experiments of FIGS. 4D, 4E and 4F are 0.5 J/cm², 5 J/cm² and 10 J/cm², respectively. The ALPase assay result is indicated by correlation of ALPase activity rate to cultivating days. The term "activity rate" specified for ALPase used herein and hereinafter indicates a ratio of averaged ALPase concentration of the gingival fibroblasts subjected to irradiation (experimental group) to that of the gingival fibroblasts receiving no irradiation (control group).

It can be seen from FIGS. 4G∼4I that the ALPase activity performance of the experimental group is apparently better than that of the control group, and the irradiation facilitates the secretion of ALPase in cells no matter whether blue, yellow or red light is used for irradiation, and no matter 0.5 J/cm² 5 J/cm² or 10 J/cm² is used as the irradiating energy. Nevertheless, it is understood that with the use of different wavelength of LED light, the optimal irradiating energy may change, and vice versa. Compared to the control group, the secretion of ALPase in cells is almost doubled.

### REGENERATION TESTS OF CELLS

In the regeneration tests of cells, the amount of proliferated cells is used as an index.

First of all, irradiation is conducted onto each of the four periodontal tissue cells, i.e. the alveolar osteoblasts, gingival fibroblasts, periodontal fibroblasts and periodontal ligmant fibroblasts, with selected wavelengths of LED light and selected irradiating energies. The three selected wavelengths of LED light include substantially 652 nm directing to red LED light, substantially 590 nm directing to yellow LED light, and substantially 415 nm directing to blue LED light. The periodontal tissue cells, after being irradiated, are cultivated for selected days and then analyzed for cell intensity. Concretely, a cell counter is used for counting cells so as to realize the cell regeneration results. The cell intensity changes of the alveolar osteoblasts, gingival fibroblasts, periodontal fibroblasts and periodontal ligmant fibroblasts are summarized in FIGS.5A∼5F and are described as follows.

In FIG. 5A, the cell regeneration of the alveolar osteoblasts, after being irradiated, is illustrated. The correlation of cell intensity rate to irradiating energy is schematically shown. In this experiment, three wavelengths of LED light, 415 nm, 590 nm, 652 nm, and three different irradiating energies, 0.5 J/cm², 5 J/cm² and 10 J/cm², are selected for use. The term "cell intensity rate" used herein and hereinafter indicates a ratio of averaged cell number of the alveolar osteoblasts subjected to irradiation (experimental group) to that of the alveolar osteoblasts receiving no irradiation (control group).

According to the experimental data, it can be seen that the cell regeneration performance of the experimental group is apparently better than that of the control group. Compared to the control group, up to 1.5 times or more of cell number in the experimental group is measured.

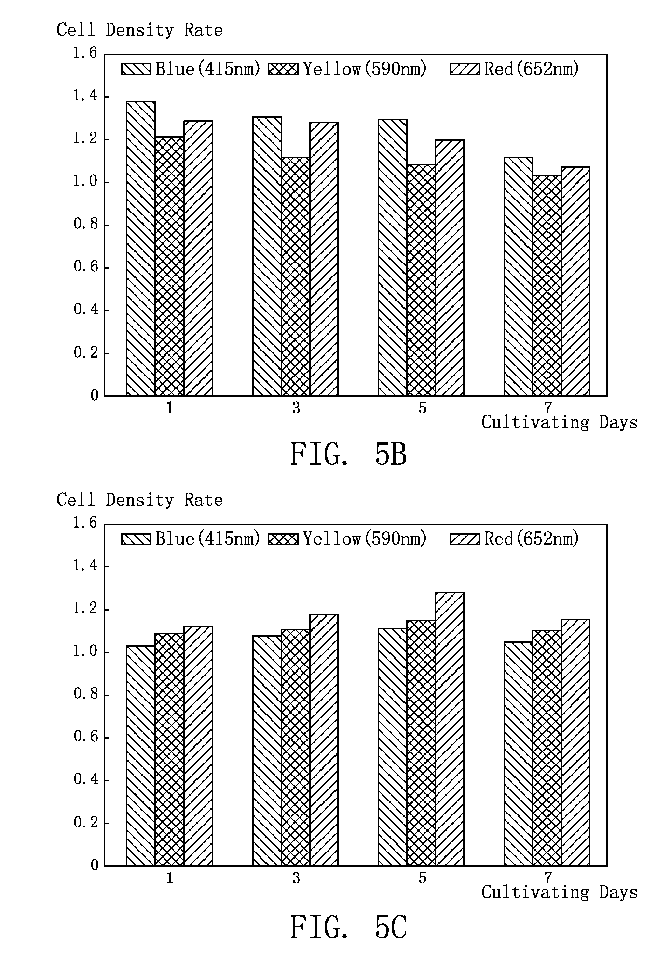

FIG. 5B and FIG. 5C reveal the cell regeneration performance of irradiated gingival fibroblasts with two different irradiating energies, respectively, by way of correlation of cell density rate to cultivation days. The irradiating energy is 0.5 J/cm² in FIG. 5B, and 5 J/cm² in FIG. 5C.

According to the experimental data, it can be seen that the cell proliferation of the experimental group is apparently better than that of the control group. Compared to the control group, up to about 1.4 times of cell number in the experimental group is obtained.

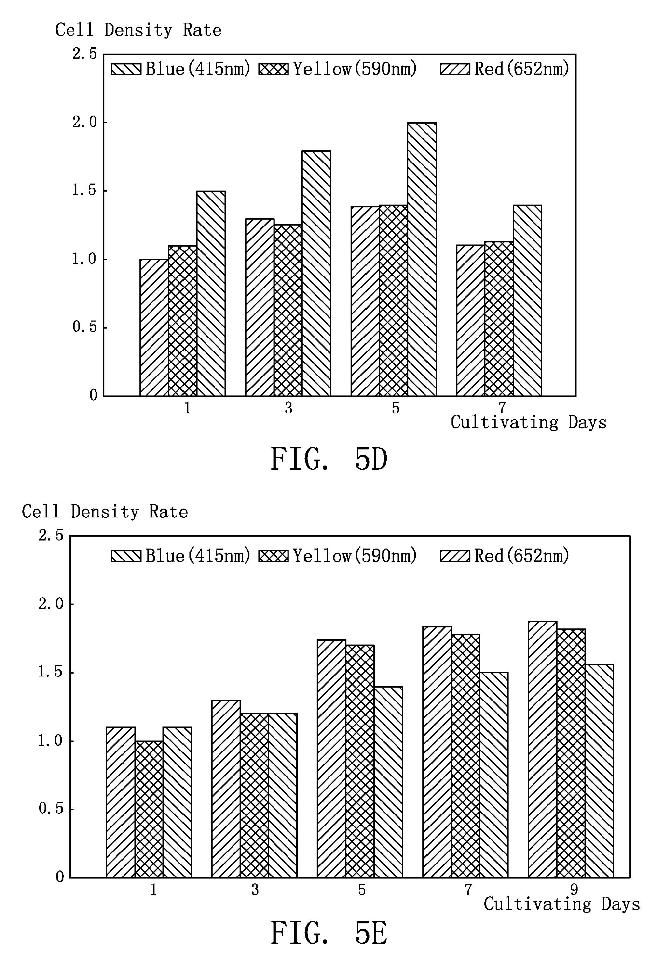

FIG. 5D and FIG. 5E reveal the cell regeneration performance of irradiated periodontal fibroblasts with two different irradiating energies, respectively, by way of correlation of cell density rate to cultivation days. The irradiating energy is 0.5 J/cm² in FIG. 5D, and 5 J/cm² in FIG. 5E.

According to the experimental data, it can be seen that the cell regeneration performance of the experimental group is apparently better than that of the control group. Compared to the control group, up to about 2 times of cell number in the experimental group is obtained.

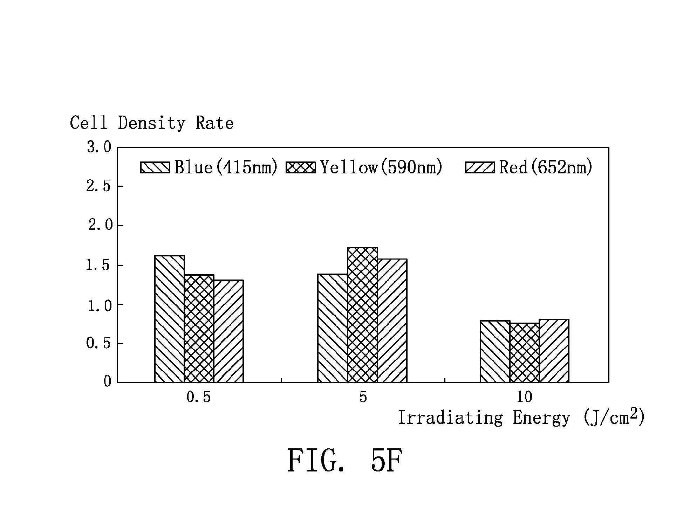

In FIG. 5F, the cell regeneration of the periodontal ligmant fibroblasts, after being irradiated, is illustrated. The correlation of cell intensity rate to irradiating energy is schematically shown. In this experiment, three wavelengths of LED light, 415 nm, 590 nm, 652 nm, and three different irradiating energies, 0.5 J/cm², 5 J/cm² and 10 J/cm², are selected for use.

According to the experimental data, it can be seen that the cell regeneration performance of the experimental group is apparently better than that of the control group. Compared to the control group, about 1.7 times of cell number in the experimental group is obtained.

In view of the above experimental results, LED irradiation conducted with an irradiating energy ranged between 0.1 J/cm² and 10 J/cm² is effective on enhancing viability and regeneration of cells. In addition, due to the low irradiating energy, the method according to the present invention is particularly suitable for daily home care use.

It is to be understood that with the use of different wavelength of LED light, the optimal irradiating energy may change, and vice versa. For example, for viability and regeneration enhancement of alveolar osteoblasts, irradiating energy between 0.2∼5 J/cm² is desirable when using blue LED light with wavelength 415±25 nm as the irradiating light source; irradiating energy between 1~10 J/cm² is desirable when using yellow LED light with wavelength 575±25 nm as the irradiating light source; and irradiating energy between 1∼10 J/cm² is desirable when using red LED light with wavelength 635±25 nm as the irradiating light source. For viability and regeneration enhancement of gingival fibroblasts, irradiating energy between 1∼5 J/cm² is desirable when using blue LED light with wavelength 415±25 nm as the irradiating light source; irradiating energy between 1~10 J/cm² is desirable when using yellow LED light with wavelength 575±25 nm as the irradiating light source; and irradiating energy between 1∼10 J/cm² is desirable when using red LED light with wavelength 635±25 nm as the irradiating light source. For viability and regeneration enhancement of periodontal fibroblasts, irradiating energy between 1∼5 J/cm² is desirable when using blue LED light with wavelength 415±25 nm as the irradiating light source; irradiating energy between 1~10 J/cm² is desirable when using yellow LED light with wavelength 575±25 nm as the irradiating light source; and irradiating energy between 1-10 J/cm² is desirable when using red LED light with wavelength 635±25 nm as the irradiating light source. For viability and regeneration enhancement of periodontal ligmant fibroblasts, irradiating energy between 0.2∼5 J/cm² is desirable when using blue LED light with wavelength 415±25 nm as the irradiating light source; irradiating energy between 0.5∼6 J/cm² is desirable when using yellow LED light with wavelength 575±25 nm as the irradiating light source; and irradiating energy between 0.5∼6 J/cm² is desirable when using red LED light with wavelength 635±25 nm as the irradiating light source.

It is to be noted that since the irradiating energy according to the present invention is relative low, the elevation of temperature resulting from the irradiation is little and has no adverse effect on cells. Furthermore, the apoptosis effect is observed, and it is found that the periodontal cells will not be damaged unless the irradiating energy exceeds 10 J/cm². It is further advantageous in daily home care use.

## Claims

1. A method for enhancing viability of periodontal tissue cells associated with more than one tooth, comprising irradiating the periodontal tissue cells simultaneously by an LED (light emitting diode) module having a light emitting range covering the associated teeth, wherein the irradiating energy is between 0.1 J/cm² and 10 J/cm².

2. The method according to claim 1 wherein the periodontal tissue cells include alveolar osteoblasts, **characterized in that** the irradiating energy is between 0.2 J/cm² and 5 J/cm² when the LED light module emits a blue light having a wavelength of 415±25 nm.

3. The method according to claim 1 wherein the periodontal tissue cells include alveolar osteoblasts, **characterized in that** the irradiating energy is between 1 J/cm² and 10 J/cm² when the LED light module emits a yellow light having a wavelength of 575±25 nm or a red light having a wavelength of 635±25 nm.

4. The method according to claim 1 wherein the periodontal tissue cells include gingival fibroblasts, **characterized in that** the irradiating energy is between 1 J/cm² and 5 J/cm² when the LED light module emits a blue light having a wavelength of 415±25 nm.

5. The method according to claim 1 wherein the periodontal tissue cells include gingival fibroblasts, **characterized in that** the irradiating energy is between 1 J/cm² and 10 J/cm² when the LED light module emits a yellow light having a wavelength of 575±25 nm or a red light having a wavelength of 635±25 nm.

6. The method according to claim 1 wherein the periodontal tissue cells include periodontal fibroblasts, and the irradiating energy is between 1 J/cm² and 5 J/cm² when the LED light module emits a blue light having a wavelength of 415±25 nm.

7. The method according to claim 1 wherein the periodontal tissue cells include periodontal fibroblasts, **characterized in that** the irradiating energy is between 1 J/cm² and 10 J/cm² when the LED light module emits a yellow light having a wavelength of 575±25 nm or a red light having a wavelength of 635±25 nm.

8. The method according to claim 1 wherein the periodontal tissue cells include periodontal ligmant fibroblasts, **characterized in that** the irradiating energy is between 0.2 J/cm² and 5 J/cm² when the LED light module emits a blue light having a wavelength of 415±25 nm.

9. The method according to claim 1 wherein the periodontal tissue cells include periodontal ligmant fibroblasts, **characterized in that** the irradiating energy is between 0.5 J/cm² and 6 J/cm² when the LED light module emits a yellow light having a wavelength of 575±25 nm or a red light having a wavelength of 635±25 nm.

10. The method according to claim 1, **characterized in that** the LED module includes a plurality of LED light sources, adjacent two of which have a partially overlapping light emitting rang so that each of the teeth irradiated by the adjacent two LED light sources is subjected to an irradiating energy ranged between 0.1 J/cm² and 10 J/cm².

11. The method according to claim 1, **characterized in that** the light emitting range has a center at a border of a gingiva.
